# FASCICULE DE BREVET EUROPEEN

(11) **EP 1 430 871 B1**
(45) Date de publication et mention de la délivrance du brevet: **21.05.2008**
(21) Numéro de dépôt: 03292685.9
(22) Date de dépôt: 28.10.2003
(51) Int. Cl.: A61Q 5/02, A61Q 19/10, A61K 8/68, A61K 8/46

(54) **Compositions cosmétiques contenant un tensioactif amphotère et un céramide et leurs utilisations**
Kosmetische Zusammensetzungen enthaltend ein amphoteres Tensid und ein Ceramid sowie ihre Verwedung
Cosmetic compositions comprising an amphoteric surfactant and a ceramide and their use

(30) Priorité: 19.12.2002 FR 0216191
(43) Date de publication de la demande: 23.06.2004
(73) Titulaire: L'ORÉAL, 75008 Paris (FR)
(72) Inventeur: Maubru, Mireille, 78400 Chatou (FR)
(74) Mandataire: Le Blainvaux Bellegarde, Françoise

(56) Documents cités:
- EP-A- 0 887 071
- EP-A- 1 093 809
- EP-A- 1 132 079

## Description

La présente invention concerne de nouvelles compositions cosmétiques comprenant dans un milieu cosmétiquement acceptable au moins un tensioactif amphotère alcoylamphohydroxyalkylsulfonate et au moins un composé de type céramide.

Il est bien connu que des cheveux qui ont été sensibilisés (i.e. abîmés et/ou fragilisés) à des degrés divers sous l'action d'agents atmosphériques ou sous l'action de traitements mécaniques ou chimiques, tels que des colorations, des décolorations et/ou des permanentes, sont souvent difficiles à démêler et à coiffer, et manquent de douceur.

En effet, sous l'action de ces agressions (agents atmosphériques, traitements mécaniques ou chimiques), les cheveux perdent une partie de leurs constituants, tels que notamment des céramides et des protéines.

Les céramides ou leurs analogues sont connus pour protéger et/ou réparer la peau et/ou les fibres capillaires des agressions des divers agents et traitements cités ci-dessus. En particulier, ils ont un effet barrière qui limitent la fuite des protéines, ils renforcent également la cohésion cuticulaire.

Compte tenu du fait que la protection et/ou le soin apporté par les céramides est d'autant plus élevé que leurs quantités présentes sur les cheveux ou sur la peau sont importantes, la demanderesse a donc cherché à améliorer la fixation (ou dépôt) des composés de type céramides sur et/ou dans le cheveu ou sur et/ou dans la peau. On a également cherché à améliorer les propriétés cosmétiques des compositions contenant un céramide.

L'invention a donc pour but de proposer des compositions cosmétiques en particulier détergentes présentant des propriétés cosmétiques améliorées, en particulier le démêlage, le lissage, la souplesse, la malléabilité et la douceur des fibres kératiniques telles que notamment les cheveux.

L'invention a donc pour but d'améliorer la fixation (ou dépôt) des composés de type céramides sur et/ou dans les matières kératiniques telles que les cheveux et/ou la peau.

Or, la demanderesse a maintenant trouvé qu'un tensioactif amphotère alcoylamphohydroxyalkylsulfonate associé à des composés de type céramide permettait d'atteindre ces buts.

Les tensioactifs amphotères alcoylamphohydroxyalkylsulfonates ont déjà été préconisés dans des compositions cosmétiques détergentes. Ils ont été décrits par exemple dans la demande de brevet WO99136054.

Les compositions de lavage des cheveux utilisant ces tensioactifs seuls ne conduisent pas à de propriétés cosmétiques satisfaisantes.

L'invention a ainsi pour objet une composition cosmétique, caractérisée en ce qu'elle comprend, dans un milieu cosmétiquement acceptable au moins un tensioactif amphotère alcoylampho hydroxyalkyl sulfonate ou ses sels et au moins un composé de type céramide.

L'invention a également pour objet l'utilisation d'un tensioactif amphotère alcoylamphohydroxyalkylsulfonate ou ses sels pour améliorer la fixation (ou le dépôt) des composés de type céramides sur et/ou dans les matières kératiniques telles que les cheveux et/ou la peau.

L'invention a encore pour objet l'utilisation d'une composition selon l'invention pour améliorer le démêlage ou le lissage des cheveux, ou pour apporter du volume, de la légèreté, de la douceur, de la souplesse ou de la malléabilité aux cheveux.

L'invention a encore pour objet l'utilisation d'un tensioactif amphotère alcoylampho hydroxyalkyl sulfonate ou ses sels dans une composition cosmétique contenant au moins un composé de type céramide pour améliorer le démêlage ou le lissage des cheveux, ou pour apporter du volume, de la légèreté, de la douceur, de la souplesse ou de la malléabilité aux cheveux.

Un autre objet de l'invention concerne un procédé de traitement des matières kératiniques, telles que les cheveux, caractérisé en ce qu'il consiste à appliquer sur lesdites matières des compositions cosmétiques selon l'invention.

Selon la présente invention, par matières kératiniques, on comprend les cheveux, les cils, les sourcils, la peau, les ongles, les muqueuses ou le cuir chevelu et plus particulièrement les cheveux.
Selon la présente invention, par fibres kératiniques, on comprend les cheveux, les cils, les sourcils, et plus particulièrement les cheveux.

Les différents objets de l'invention vont maintenant être détaillés. L'ensemble des significations et définitions des composés utilisés dans la présente invention données ci-dessous sont valables pour l'ensemble des objets de l'invention.

Selon la présente invention, on entend, par composé de type céramide, les céramides et/ou les glycocéramides et/ou les pseudocéramides et/ou les néocéramides, naturels ou synthétiques.

Des composés de type céramides sont par exemple décrits dans les demandes de brevet DE4424530, DE4424533, DE4402929, DE4420736, WO95/23807, EP-A-0646572, WO95/16665, FR-2 673 179, EP-A-0227994 et WO 94/07844, WO94/24097, WO94/10131.

Les composés de type céramide utilisables selon la présente invention répondent par exemple à la formule générale (I) suivante : 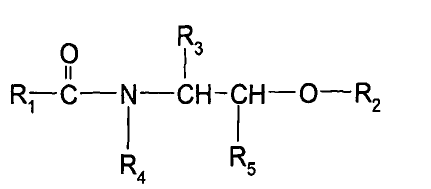 dans laquelle :
- R₁ désigne :

- soit un radical hydrocarboné, linéaire ou ramifié, saturé ou insaturé, en C₁-C₅₀, de préférence en C₅-C₅₀, ce radical pouvant être substitué par un ou plusieurs groupements hydroxyle éventuellement estérifié par un acide R₇COOH, R₇ étant un radical hydrocarboné, saturé ou insaturé, linéaire ou ramifié, éventuellement mono ou polyhydroxylé, en C₁-C₃₅ , le ou les hydroxyles du radical R₇ pouvant être estérifié par un acide gras saturé ou insaturé, linéaire ou ramifié, éventuellement mono ou polyhydroxylé, en C₁-C₃₅;
- soit un radical R"-(NR°-CO)-R', R° désigne un atome d'hydrogène ou un radical hydrocarboné C₁-C₂₀ mono ou polyhydroxylé, préférentiellement monohydroxylé, R' et R" sont des radicaux hydrocarbonés dont la somme des atomes de carbone va de 9 à 30, R' étant un radical divalent.
- soit un radical R₈-O-CO-(CH2)ₚ, R₈ désigne un radical hydrocarboné en C₁-C₂₀, p est un entier variant de 1 à 12.
- R₂ est choisi parmi un atome d'hydrogène, un radical de type saccharidique, en particulier un radical (glycosyle)ₙ, (galactosyle)ₘ ou sulfogalactosyle, un résidu de sulfate ou de phosphate, un radical phosphoryléthylamine et un radical phosphoryléthylammonium, dans lesquels n est un entier variant de 1 à 4 et m est un entier variant de 1 à 8 ;
- R₃ désigne un atome d'hydrogène ou un radical hydrocarboné en C₁-C₃₃, saturé ou insaturé, mono ou polyhydroxylé ou non hydroxylé, le ou les hydroxyles pouvant être estérifiés par un acide minéral ou un acide R₇COOH, R₇ ayant les mêmes significations que ci-dessus, le ou les hydroxyles pouvant être éthérifiés par un radical (glycosyle)ₙ, (galactosyle)ₘ, sulfogalactosyle, phosphoryléthylamine ou phosphoryléthylammonium, R₃ pouvant également être substitué par un ou plusieurs radicaux alkyle en C₁-C₁₄ ;
   de préférence, R₃ désigne un radical α-hydroxyalkyle en C₁₅-C₂₆, le groupement hydroxyle étant éventuellement estérifié par un α-hydroxyacide en C₁₆-C₃₀ ;
- R₄ désigne un atome d'hydrogène, un radical méthyle, éthyle, un radical hydrocarboné en C₃-C₅₀, saturé ou insaturé, linéaire ou ramifié, éventuellement hydroxylé ou un radical -CH₂-CHOH-CH₂-O-R₆ dans lequel R₆ désigne un radical hydrocarboné en C₁₀-C₂₆ ou un radical R₈-O-CO-(CH2)ₚ, R₈ désigne un radical hydrocarboné en C₁-C₂₀, p est un entier variant de 1 à 12,
- R₅ désigne un atome d'hydrogène ou un radical hydrocarboné en C₁-C₃₀ saturé ou insaturé, linéaire ou ramifié, éventuellement mono ou polyhydroxylé, le ou les hydroxyles pouvant être éthérifiés par un radical (glycosyle)ₙ, (galactosyle)ₘ, sulfogalactosyle, phosphoryléthylamine ou phosphoryléthylammonium,
sous réserve que lorsque R₃ et R₅ désignent hydrogène ou lorsque R₃ désigne hydrogène et R₅ désigne méthyle alors R₄ ne désigne pas un atome d'hydrogène, un radical méthyle ou éthyle.

Parmi les composés de formule (I), on préfère les céramides et/ou glycocéramides dont la structure est décrite par DOWNING dans Journal of Lipid Research Vol. 35, 2060-2068, 1994, ou ceux décrits dans la demande de brevet français FR-2 673 179.

Les composés de type céramide plus particulièrement préférés selon l'invention sont les composés de formule (I) pour lesquels R₁ désigne un alkyle saturé ou insaturé dérivé d'acides gras en C₁₄-C₂₂ éventuellement hydroxylé; R₂ désigne un atome d'hydrogène ; et R₃ désigne un radical linéaire en C₁₁₋₁₇ éventuellement hydroxylé et de préférence en C₁₃₋₁₅.

De tels composés sont par exemple :
- le 2-N-linoléoylamino-octadécane-1,3-diol,
- le 2-N-oléoylamino-octadécane-1,3-diol,
- le 2-N-palmitoylamino-octadécane-1,3-diol,
- le 2-N-stéaroylamino-octadécane-1,3-diol,
- le 2-N-béhénoylamino-octadécane-1,3-diol,
- le 2-N-[2-hydroxy-palmitoyl]-amino-octadécane-1,3-diol,
- le 2-N-stéaroyl amino-octadécane-1,3,4 triol et en particulier la N-stéaroyl phytosphingosine,
- le 2-N-palmitoylamino-hexadécane-1,3-diol
ou les mélanges de ces composés.

On peut également utiliser les composés de formule (I) pour lesquels R₁ désigne un radical alkyle saturé ou insaturé dérivé d'acides gras en C₁₂-C₂₂ ; R₂ désigne un radical galactosyle ou sulfogalactosyle ; et R₃ désigne un radical hydrocarboné en C₁₂-C₂₂, saturé ou insaturé et de préférence un groupement CH(OH)-CH=CH-(CH₂)₁₂₋CH₃.

A titre d'exemple, on peut citer le produit constitué d'un mélange de glycocéramides, vendu sous la dénomination commerciale GLYCOCER par la société WAITAKI INTERNATIONAL BIOSCIENCES.

On peut également utiliser les composés de formule (I) décrits dans les demandes de brevet EP-A-0227994 et WO 94/07844.

De tels composés sont par exemple le QUESTAMIDE H (bis-(N-hydroxyéthyl N-cétyl) malonamide) vendu par la société QUEST.

On peut également utiliser le N-docosanoyl N-méthyl-D-glucamine décrit dans la demande de brevet WO94/24097.

La concentration en composés de type céramide peut varier entre 0,0001% et 20% en poids environ par rapport au poids total de la composition, et de préférence entre 0,001 et 10% environ et encore plus préférentiellement entre 0,005 et 3 % en poids.

Les tensioactifs amphotères alcoylamphohydroxyalkylsulfonates peuvent avoir la formule suivante (II) : 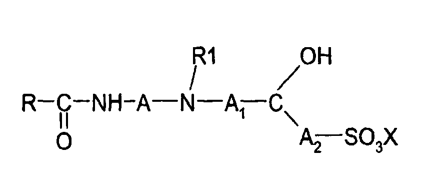 dans laquelle :
R désigne un radical hydrocarboné, saturé ou insaturé, linéaire ou ramifié comportant de 5 à 29 atomes de carbone.
R désigne de préférence un radical alkyle ou alkényle mono ou polyinsaturé comportant de 5 à 29 atomes de carbone et de préférence de 7 à 22 atomes de carbone et encore plus particulièrement de 9 à 17 atomes de carbone.
R1 désigne un radical hydroxyalkyle en C₁-C₄, de préférence hydroxyéthyle,
A, A1, A2 , identiques ou différents, désignent un radical alkylène divalent linéaire ou ramifié en C₁-C₁₀, de préférence en C₁-C₃
X désigne un atome d'hydrogène, un cation minéral ou organique tel que: celui d'un métal alcalin (par exemple Na⁺, K⁺) , celui d'un métal alcalinoterreux (Mg²⁺, Ca²⁺) , l'ion NH₄⁺ , les ions ammoniums issus des aminoacides basiques ou des amino-alcools.

Des composés préférés selon la présente invention sont des composés de formule (II) dans laquelle R désigne plus particulièrement un radical alkyle saturé, linéaire ou ramifié comportant de 7 à 29 atomes de carbone, de préférence de 7 à 22 atomes de carbone.

Lorsque X désigne un ion ammonium issu d'alcanolamine, celle-ci peut être la monoéthanolamine, la diéthanolamine, la triéthanolamine, l'amino-3 propanediol-1,2. Lorsque X désigne un ion ammonium issu d'amine, cette amine peut être un aminoacide basique tel que la lysine, l'arginine, la sarcosine, l'ornithine ou la citrulline.

De préférence, A= A2 et désigne -CH₂CH₂-.
De préférence, A1 désigne -CH₂-
De préférence X désigne Na⁺

Parmi les tensioactifs de formule (II), on peut citer plus particulièrement les sels de Cocoyl amphohydroxypropyl sulfonate et notamment le sel de sodium tel que le produit proposé sous la dénomination MIRANOL CSE par la société RHODIA CHIMIE ou encore les sels de palmitoyl amphohydroxypropyl sulfonate.

Selon l'invention, le ou les tensioactifs amphotères choisis parmi les composés alcoylamphohydroxyalkylsulfonates et leurs sels peuvent représenter de 0,1 % à 30 % en poids, de préférence de 1 % à 20% en poids et plus particulièrement de 1,5 % à 15 % en poids, plus particulièrement de 2 à 8% par rapport au poids total de la composition finale.

Les compositions de l'invention contiennent en outre avantageusement au moins un autre agent tensioactif qui est généralement présent en une quantité comprise entre 0,1% et 60% en poids environ, de préférence entre 3% et 40% et encore plus préférentiellement entre 5% et 30%, par rapport au poids total de la composition.

Cet agent tensioactif peut être choisi parmi les agents tensioactifs anioniques, amphotères, non-ioniques, cationiques ou leurs mélanges.

Les tensioactifs additionnels convenant à la mise en oeuvre de la présente invention sont notamment les suivants :

### (i) Tensioactif(s) anionique(s):

Leur nature ne revêt pas, dans le cadre de la présente invention, de caractère véritablement critique.
Ainsi, à titre d'exemple de tensioactifs anioniques utilisables, seuls ou mélanges, dans le cadre de la présente invention, on peut citer notamment (liste non limitative) les sels (en particulier sels alcalins, notamment de sodium, sels d'ammonium, sels d'amines, sels d'aminoalcools ou sels de magnésium) des composés suivants : les alkylsulfates, les alkyléthersulfates, alkylamidoéthersulfates, alkylarylpolyéthersulfates, monoglycérides sulfates ; les alkylsulfonates, alkylphosphates, alkylamidesulfonates, alkylarylsulfonates, α-oléfine-sulfonates, paraffine-sulfonates ; les alkylsulfosuccinates, les alkyléthersulfosuccinates, les alkylamidesulfosuccinates; les alkylsulfosuccinamates ; les alkylsulfoacétates ; les alkylétherphosphates; les acylsarcosinates ; les acyliséthionates et les N-acyltaurates, le radical alkyle ou acyle de tous ces différents composés comportant de préférence de 8 à 24 atomes de carbone, et le radical aryl désignant de préférence un groupement phényle ou benzyle. Parmi les tensioactifs anioniques encore utilisables, on peut également citer les sels d'acides gras tels que les sels des acides oléique, ricinoléique, palmitique, stéarique, les acides d'huile de coprah ou d'huile de coprah hydrogénée ; les acyl-lactylates dont le radical acyle comporte 8 à 20 atomes de carbone. On peut également utiliser des tensioactifs faiblement anioniques, comme les acides d'alkyl D galactoside uroniques et leurs sels ainsi que les acides alkyl (C₆-C₂₄) éther carboxyliques polyoxyalkylénés, les acides alkyl(C₆-C₂₄)aryl éther carboxyliques polyoxyalkylénés ,les acides alkyl(C₆-C₂₄) amido éther carboxyliques polyoxyalkylénés et leurs sels, en particulier ceux comportant de 2 à 50 groupements oxyde d'éthylène, et leurs mélanges.

On utilise de préférence un agent tensioactif anionique choisi parmi les alkyl(C₁₂-C₁₄) sulfates de sodium, de magnésium ou d'ammonium, les alkyl (C₁₂-C₁₄)éthersulfates de sodium, de magnésium ou d'ammonium oxyéthylénés à 2,2 moles d'oxyde d'éthylène, le cocoyl iséthionate de sodium et leurs mélange.

Parmi les tensioactifs anioniques, on préfère utiliser selon l'invention les sels d'alkylsulfates et d'alkyléthersufates et leurs mélanges.

### (ii) Tensioactif(s) non ionique(s) :

Les agents tensioactifs non-ioniques sont, eux aussi, des composés bien connus en soi (voir notamment à cet égard "Handbook of Surfactants" par M.R. PORTER, éditions Blackie & Son (Glasgow and London), 1991, pp 116-178) et leur nature ne revêt pas, dans le cadre de la présente invention, de caractère critique. Ainsi, ils peuvent être notamment choisis parmi (liste non limitative) les alcools, les alpha-diols, les alkylphénols ou les acides gras polyéthoxylés, polypropoxylés ou polyglycérolés, ayant une chaîne grasse comportant par exemple 8 à 18 atomes de carbone, le nombre de groupements oxyde d'éthylène ou oxyde de propylène pouvant aller notamment de 2 à 50 et le nombre de groupements glycérol pouvant aller notamment de 2 à 30. On peut également citer les copolymères d'oxyde d'éthylène et de propylène, les condensats d'oxyde d'éthylène et de propylène sur des alcools gras ; les amides gras polyéthoxylés ayant de préférence de 2 à 30 moles d'oxyde d'éthylène, les amides gras polyglycérolés comportant en moyenne 1 à 5 groupements glycérol et en particulier 1,5 à 4 ; les esters d'acides gras du sorbitan oxyéthylénés ayant de 2 à 30 moles d'oxyde d'éthylène ; les esters d'acides gras du sucrose, les esters d'acides gras du polyéthylèneglycol, les alkylpolyglycosides, les dérivés de N-alkyl glucamine, les oxydes d'amines tels que les oxydes d'alkyl (C₁₀ - C₁₄) amines ou les oxydes de N-acylaminopropylmorpholine. On notera que les alkylpolyglycosides constituent des tensioactifs non-ioniques rentrant particulièrement bien dans le cadre de la présente invention.

### (iii) Tensioactif(s) amphotère(s):

Les agents tensioactifs amphotères additionnels, dont la nature ne revêt pas dans le cadre de la présente invention de caractère critique, peuvent être notamment (liste non limitative) des dérivés d'amines secondaires ou tertiaires aliphatiques, dans lesquels le radical aliphatique est une chaîne linéaire ou ramifiée comportant 8 à 22 atomes de carbone et contenant au moins un groupe anionique hydrosolubilisant (par exemple carboxylate, sulfonate, sulfate, phosphate ou phosphonate) ; on peut citer encore les alkyl (C₈-C₂₀) bétaïnes, les sulfobétaïnes, les alkyl (C₈-C₂₀) amidoalkyl (C₁-C₆) bétaïnes ou les alkyl (C₈-C₂₀) amidoalkyl (C₁-C₆) sulfobétaïnes.

Parmi les dérivés d'amines, on peut citer les produits commercialisés sous les dénomination MIRANOL, tels que décrits dans les brevets US-2 528 378 et US-2 781 354 et de structures :

R₂ -CONHCH₂CH₂ -N(R₃)(R₄)(CH₂COO-) (2)

dans laquelle : R₂ désigne un radical alkyle dérivé d'un acide R₂-COOH présent dans l'huile de coprah hydrolysée, un radical heptyle, nonyle ou undécyle, R₃ désigne un groupement bêta-hydroxyéthyle et R₄ un groupement carboxyméthyle ;
et

R₅-CONHCH₂CH₂-N(B)(C) (3)

dans laquelle :
B représente -CH₂CH₂OX', C représente -(CH₂)_{z} -Y', avec z = 1 ou 2,
X' désigne le groupement -CH₂CH₂-COOH ou un atome d'hydrogène
Y' désigne -COOH ou le radical -CH₂ - CHOH - SO3H
R₅ désigne un radical alkyle d'un acide R₉ -COOH présent dans l'huile de coprah ou dans l'huile de lin hydrolysée, un radical alkyle, notamment en C₇, C₉, C₁₁ ou C₁₃, un radical alkyle en C₁₇ et sa forme iso, un radical C₁₇ insaturé.

Ces composés sont classés dans le dictionnaire CTFA, 5ème édition, 1993, sous les dénominations Disodium Cocoamphodiacetate, Disodium Lauroamphodiacetate, Disodium Caprylamphodiacetate, Disodium Capryloamphodiacetate, Disodium Cocoamphodipropionate, Disodium Lauroamphodipropionate, Disodium Caprylamphodipropionate, Disodium Capryloamphodipropionate, Lauroamphodipropionic acid, Cocoamphodipropionic acid.
A titre d'exemple on peut citer le cocoamphodiacetate commercialisé sous la dénomination commerciale MIRANOL^{®} C2M concentré par la société RHODIA CHIMIE.

Dans les compositions conformes à l'invention, on utilise de préférence des mélanges d'agents tensioactifs et en particulier des mélanges d'agents tensioactifs anioniques et des mélanges d'agents tensioactifs anioniques et d'agents tensioactifs amphotères ou non ioniques. Un mélange particulièrement préféré est un mélange constitué d'au moins un agent tensioactif anionique et d'au moins un agent tensioactif amphotère.

On peut également utiliser des tensioactifs cationiques parmi lesquels on peut citer en particulier (liste non limitative) : les sels d'amines grasses primaires, secondaires ou tertiaires, éventuellement polyoxyalkylénées ; les sels d'ammonium quaternaire tels que les chlorures ou les bromures de tétraalkylammonium, d'alkylamidoalkyltrialkylammonium, de trialkylbenzylammonium, de trialkylhydroxyalkyl-ammonium ou d'alkylpyridinium; les dérivés d'imidazoline ; ou les oxydes d'amines à caractère cationique.

Les compositions conformes à l'invention peuvent contenir en plus de l'association définie ci-dessus des agents régulateurs de viscosité tels que des électrolytes, ou des agents épaississants. On peut citer en particulier le chlorure de sodium, le xylène sulfonate de sodium, les scléroglucanes, les gommes de xanthane, les alcanolamides d'acide gras, les alcanolamides d'acide alkyl éther carboxylique éventuellement oxyéthylénés avec jusqu'à 5 moles d'oxyde d'éthylène tel que le produit commercialisé sous la dénomination "AMINOL A15" par la société CHEM Y, les acides polyacryliques réticulés et les copolymères acide acrylique / acrylates d'alkyle en C₁₀-C₃₀ réticulés. Ces agents régulateurs de viscosité sont utilisés dans les compositions selon l'invention dans des proportions pouvant aller jusqu'à 10 % en poids par rapport au poids total de la composition.

Les compositions conformes à l'invention peuvent également contenir jusqu'à 5 % d'agents nacrants ou opacifiants bien connus dans l'état de la technique tels que par exemple les oxydes de titane enrobés ou non, les palmitates de sodium ou de magnésium, les stéarates et hydroxystéarates de sodium ou de magnésium, les dérivés acylés à chaîne grasse tels que les monostéarates ou distéarates d'éthylène glycol ou de polyéthylèneglycol, les éthers à chaînes grasses ( par exemple en C8-C30) tels que par exemple le distéaryléther ou le 1-(hexadécyloxy)-2-octadécanol, les cyclodextrines, les alcools gras tels que les alcools cétylique, stéarylique et béhénylique.

Les compositions selon l'invention comprennent de préférence en outre au moins un ou plusieurs polymères cationiques.
Les polymères cationiques utilisables conformément à la présente invention peuvent être choisis parmi tous ceux déjà connus en soi comme améliorant les propriétés cosmétiques des cheveux traités par des compositions détergentes, à savoir notamment ceux décrits dans la demande de brevet EP-A-0 337 354 et dans les demandes de brevets français FR-A-2 270 846, 2 383 660, 2 598 611, 2 470 596 et 2 519 863.

De manière encore plus générale, au sens de la présente invention, l'expression "polymère cationique" désigne tout polymère contenant des groupements cationiques et/ou des groupements ionisables en groupements cationiques.

Parmi tous les polymères cationiques susceptibles d'être utilisés dans le cadre de la présente invention, on préfère mettre en oeuvre les dérivés d'éther de cellulose quaternaires tels que les produits commercialisés sous la dénomination « JR 400 » par la société AMERCHOL, les cyclopolymères, en particulier les homopolymères de sel de diallyldiméthylammonium et les copolymères de sel de diallyldiméthylammonium et d'acrylamide en particulier les chlorures, commercialisés sous les dénominations « MERQUAT 100 », « MERQUAT 550 » et « MERQUAT S » par la société ONDEO, les polysaccharides cationiques non cellulosiques et plus particulièrement les gommes de guar modifiées par du chlorure de 2,3-époxypropyl triméthylammonium commercialisées par exemple sous la dénomination « JAGUAR C13S » par la société RHODIA CHIMIE.

Selon l'invention, le ou les polymères cationiques peuvent représenter de 0,001 % à 10 % en poids, de préférence de 0,005 % à 5 % en poids, et encore plus préférentiellement de 0,01 % à 3 % en poids, du poids total de la composition finale.

Les compositions selon l'invention peuvent contenir également des synergistes de mousses tels que des 1,2-alcanediols en C₁₀-C₁₈ ou des alcanolamides gras dérivés de mono ou de diéthanolamine.

La composition de l'invention peut également contenir au moins un additif choisi parmi les parfums, les conservateurs, les filtres solaires, les polymères anioniques ou non ioniques ou amphotères, les protéines, les hydrolysats de protéines, les acides gras à chaînes linéaires ou ramifiées en C₁₆-C₄₀ tels que l'acide méthyl-18 eicosanoique, les hydroxyacides, les vitamines, le panthénol, les huiles végétales, animales, minérales ou synthétiques, les silicones volatiles ou non volatiles, les esters gras et tout autre additif classiquement utilisé dans le domaine cosmétique qui n'affecte pas les propriétés des compositions selon l'invention.

Ces additifs sont présents dans la composition selon l'invention dans des proportions pouvant aller de 0 à 50% en poids par rapport au poids total de la composition. La quantité précise de chaque additif est déterminée facilement par l'homme du métier selon sa nature et sa fonction.

Le milieu cosmétiquement acceptable peut être constitué uniquement par de l'eau
ou par un mélange d'eau et d'un solvant cosmétiquement acceptable tel qu'un alcool inférieur en C₁-C₄, comme l'éthanol, l'isopropanol, le tertiobutanol, le n-butanol ; les alkylèneglycols comme le propylèneglycol, les éthers de glycols.
De préférence , la composition comprend de 50 à 95 % en poids d'eau par rapport au poids total de la composition.

Les compositions selon l'invention présentent un pH final généralement compris entre 3 et 10. De préférence, ce pH est compris entre 4 et 8. L'ajustement du pH à la valeur désirée peut se faire classiquement par ajout d'une base (organique ou minérale) dans la composition, par exemple de l'ammoniaque ou une (poly)amine primaire, secondaire ou tertiaire comme la monoéthanolamine, la diéthanolamine, la triéthanolamine, l'isopropanolamine ou la propanediamine-1,3, ou encore par ajout d'un acide, de préférence un acide carboxylique tel que par exemple l'acide citrique.

Les compositions conformes à l'invention peuvent être utilisées pour le lavage ou le traitement des matières kératiniques telles que les cheveux, la peau, les cils, les sourcils, les ongles, les lèvres, le cuir chevelu et plus particulièrement les cheveux.

En particulier, les compositions selon l'invention sont des compositions détergentes telles que des shampooings, des gels-douche et des bains moussants.
Dans ce mode de réalisation de l'invention, les compositions comprennent une base lavante, généralement aqueuse. La base lavante comprend d'autres tensioactifs additionnels.

Le ou les tensioactifs additionnels peuvent être choisis, seuls ou en mélanges, dé préférence au sein des tensioactifs anioniques, non ioniques tels que définis ci-dessus.

La quantité et la qualité de la base lavante sont celles suffisantes pour conférer à la composition finale un pouvoir moussant et/ou détergent satisfaisant.

Ces compositions détergentes sont de préférence moussante et le pouvoir moussant des compositions selon l'invention, caractérisé par une hauteur de mousse, est généralement supérieur à 75 mm ; de préférence, supérieure à 100 mm mesurée selon la méthode ROSS-MILES (NF T 73-404 /ISO696) modifiée.
Les modifications de la méthode sont les suivantes :
La mesure se fait à la température de 22°C avec de l'eau osmosée. La concentration de la solution est de 2g/l. La hauteur de la chute est de 1 m. La quantité de composition qui chute est de 200 ml. Ces 200 ml de composition tombe dans une éprouvette ayant un diamètre de 50 mm et contenant 50 ml de la composition à tester. La mesure est faite 5 minutes après l'arrêt de l'écoulement de la composition.

Ainsi, selon l'invention, la base lavante peut représenter de 3 % à 50 % en poids, de préférence de 6 % à 35 % en poids, et encore plus préférentiellement de 8 % à 25 % en poids, du poids total de la composition finale.

L'invention a encore pour objet un procédé de traitement des matières kératiniques telles que la peau ou les cheveux, caractérisé en ce qu'il consiste à appliquer sur les matières kératiniques une composition cosmétique telle que définie précédemment, puis à effectuer éventuellement un rinçage à l'eau.

Ainsi, ce procédé selon l'invention permet le maintien de la coiffure, le traitement, le soin, le lavage ou le démaquillage de la peau, des cheveux ou de toute autre matière kératinique.

Les compositions de l'invention peuvent également se présenter sous forme d'après-shampooing à rincer ou non, de compositions pour permanente, défrisage, coloration ou décoloration, ou encore sous forme de compositions à rincer, à appliquer avant ou après une coloration, une décoloration, une permanente ou un défrisage ou encore entre les deux étapes d'une permanente ou d'un défrisage.

Les compositions de l'invention peuvent encore se présenter sous la forme de compositions lavantes pour la peau, et en particulier sous la forme de solutions ou de gels pour le bain ou la douche ou de produits démaquillants.

Les compositions selon l'invention peuvent se présenter sous forme de lotions aqueuses ou hydroalcooliques, de gel, de lait, de crème, d'émulsion, de lotion épaissie ou de mousse et être utilisées pour la peau, les ongles, les cils, les lèvres et plus particulièrement les cheveux.

Dans tout ce qui suit ou ce qui précède, les pourcentages exprimés sont en poids.

L'invention va être maintenant plus complètement illustrée à l'aide des exemples suivants qui ne sauraient être considérés comme la limitant aux modes de réalisation décrits.
Dans les exemples, MA signifie matière active.

### EXEMPLE 1

On a réalisé une composition de shampooing conforme à l'invention :

| Composition | Exemple 1 | |
|---|---|---|
| Lauryléthersulfate de sodium (C12/C14 à | 15,5 | g M.A. |
| 70/30) à 2.2 moles d'oxyde d'éthylène | | |
| Cocoyl amphohydroxypropyl sulfonate de | 2,2 | g M.A. |
| sodium, en solution aqueuse à 32% de | | |
| matière active, proposé sous la dénomination | | |
| Miranol CSE par la société RHODIA | | |
| Gomme de guar modifiée par du chlorure de | 0,1 | g |
| 2,3-époxypropyl triméthyl ammonium | | |
| vendue sous la dénomination Jaguar C13 S | | |
| par la société RHODIA | | |
| 2-N-oléoylamino-octadécane-1,3-diol | 0,01 | g |
| Polydiméthylsiloxane de viscosité 300 000 | 2,7 | g |
| cSt, vendu sous la dénomination DC 200 fluid | | |
| 300 000 par la société DOW CORNING | | |
| Distéaryl éther | 1,5 | g |
| Alcool béhénylique | 1,5 | g |
| Monoisopropanolamide d'acides de coprah | 1 | g |
| Acide polyacrylique réticulé | 0.2 | g |
| Conservateurs | q.s. | |
| Parfum | q.s. | |
| Acide citrique q.s. | pH | 7 |
| Eau déminéralisée q.s.p. | 100 | g |

On effectue un shampooing en appliquant environ 12 g de la composition sur des cheveux préalablement mouillés. On fait mousser le shampooing puis on rince abondamment à l'eau.
Les cheveux lavés avec ces compositions se démêlent facilement, sont lisses au toucher de la racine à la pointe, et malléables (souples).

### EXEMPLE 2

On a réalisé une composition de shampooing conforme à l'invention :

| Composition | | |
|---|---|---|
| Lauryléthersulfate de sodium (C12/C14 à | 10 | g M.A. |
| 70/30) à 2.2 moles d'oxyde d'éthylène | | |
| Cocoyl amphohydroxypropyl sulfonate de | 3,2 | g M.A. |
| sodium, en solution aqueuse à 32% de | | |
| matière active, proposé sous la dénomination | | |
| Miranol CSE par la société RHODIA | | |
| Hydroxyéthylcellulose réticulée à | 0,3 | g |
| l'épichlorhydrine, quaternisée par la | | |
| triméthylamine vendue sous la dénomination | | |
| JR400 par la société AMERCHOL | | |
| Bis-(N-hydroxyéthyl N-cétyl) malonamide | 0,05 | g |
| commercialisé sous la dénomination | | |
| Questamide H par la société QUEST | | |
| Monoisopropanolamide d'acides de coprah | 2,5 | g |
| Conservateurs | q.s. | |
| Parfum | q.s. | |
| Acide citrique q.s. | pH | 7 |
| Eau déminéralisée q.s.p. | 100 | g |

## Revendications

1. Composition cosmétique, **caractérisée par le fait qu'**elle comprend, dans un milieu cosmétiquement acceptable, au moins un tensioactif amphotère choisi parmi les composés alcoylamphohydroxyalkylsulfonates et leurs sels et au moins un composé de type céramide.

2. Composition selon la revendication 1, **caractérisée en ce que** le composé de type céramide est de formule (I) suivante : 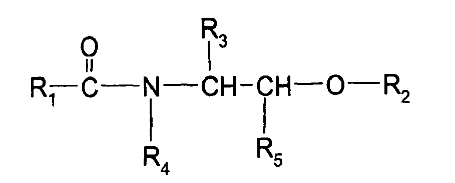 dans laquelle :
- R₁ désigne :
- soit un radical hydrocarboné, linéaire ou ramifié, saturé ou insaturé, en C₁-C₅₀, de préférence en C₅-C₅₀, ce radical pouvant être substitué par un ou plusieurs groupements hydroxyle éventuellement estérifié par un acide R₇COOH, R₇ étant un radical hydrocarboné, saturé ou insaturé, linéaire ou ramifié, éventuellement mono ou polyhydroxylé, en C₁-C₃₅ , le ou les hydroxyles du radical R₇ pouvant être estérifié par un acide gras saturé ou insaturé, linéaire ou ramifié, éventuellement mono ou polyhydroxylé, en C₁-C₃₅;
- soit un radical R"-(NR°-CO)-R', R° désigne un atome d'hydrogène ou un radical hydrocarboné C₁-C₂₀ mono ou polyhydroxylé, préférentiellement monohydroxylé, R' et R" sont des radicaux hydrocarbonés dont la somme des atomes de carbone va de 9 à 30, R' étant un radical divalent.
- soit un radical R₈-O-CO-(CH2)ₚ, R₈ désigne un radical hydrocarboné en C₁-C₂₀, p est un entier variant de 1 à 12.
- R₂ est choisi parmi un atome d'hydrogène, un radical de type saccharidique, en particulier un radical (glycosyle)ₙ, (galactosyle)ₘ ou sulfogalactosyle, un résidu de sulfate ou de phosphate, un radical phosphoryléthylamine et un radical phosphoryléthylammonium, dans lesquels n est un entier variant de 1 à 4 et m est un entier variant de 1 à 8 ;
- R₃ désigne un atome d'hydrogène ou un radical hydrocarboné en C₁-C₃₃, saturé ou insaturé, mono ou polyhydroxylé ou non hydroxylé, le ou les hydroxyles pouvant être estérifiés par un acide minéral ou un acide R₇COOH, R₇ ayant les mêmes significations que ci-dessus, le ou les hydroxyles pouvant être éthérifiés par un radical (glycosyle)ₙ, (galactosyle)ₘ, sulfogalactosyle, phosphoryléthylamine ou phosphoryléthylammonium, R₃ pouvant également être substitué par un ou plusieurs radicaux alkyle en C₁-C₁₄ ;
de préférence, R₃ désigne un radical α-hydroxyalkyle en C₁₅-C₂₆, le groupement hydroxyle étant éventuellement estérifié par un α-hydroxyacide en C₁₆-C₃₀;
- R₄ désigne un atome d'hydrogène, un radical méthyle, éthyle, un radical hydrocarboné en C₃-C₅₀, saturé ou insaturé, linéaire ou ramifié, éventuellement hydroxylé ou un radical -CH₂-CHOH-CH₂-O-R₆ dans lequel R₆ désigne un radical hydrocarboné en C₁₀-C₂₆ ou un radical R₈-O-CO-(CH2)ₚ, R₈ désigne un radical hydrocarboné en C₁-C₂₀, p est un entier variant de 1 à 12,
- R₅ désigne un atome d'hydrogène ou un radical hydrocarboné en C₁-C₃₀ saturé ou insaturé, linéaire ou ramifié, éventuellement mono ou polyhydroxylé, le ou les hydroxyles pouvant être éthérifiés par un radical (glycosyle)ₙ, (galactosyle)ₘ, sulfogalactosyle, phosphoryléthylamine ou phosphoryléthylammonium,
sous réserve que lorsque R₃ et R₅ désignent hydrogène ou lorsque R₃ désigne hydrogène et R₅ désigne méthyle alors R₄ ne désigne pas un atome d'hydrogène, un radical méthyle ou éthyle.

3. Composition selon l'une quelconque des revendications 1 ou 2, **caractérisée par le fait que** le composé de type céramide est choisi dans le groupe constitué par:
- le 2-N-linoléoylamino-octadécane-1,3-diol,
- le 2-N-oléoylamino-octadécane-1,3-diol,
- le 2-N-palmitoylamino-octadécane-1,3-diol,
- le 2-N-stéaroylamino-octadécane-1,3-diol,
- le 2-N-béhénoylamino-octadécane-1,3-diol,
- le 2-N-[2-hydroxy-palmitoyl]-amino-octadécane-1,3-diol,
- le 2-N-stéaroyl amino-octadécane-1,3,4 triol et en particulier la N-stéaroyl phytosphingosine,
- le 2-N-palmitoylamino-hexadécane-1,3-diol
- le (bis-(N-hydroxyéthyl N-cétyl) malonamide),
- le N-(2-hydroxyéthyl)-N-(3-cétyloxy-2-hydroxypropyl)amide d'acide cétylique .
- le N-docosanoyl N-méthyl-D-glucamine
ou les mélanges de ces composés.

4. Composition selon l'une quelconque des revendications 1 à 3, **caractérisée par le fait que** ledit tensioactif amphotère est choisi parmi les composés alcoylamphohydroxyalkylsulfonates pouvant avoir la formule suivante (I) : 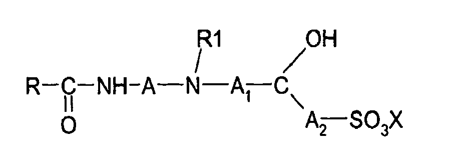 dans laquelle :
R désigne un radical hydrocarboné, saturé ou insaturé, linéaire ou ramifié comportant de 5 à 29 atomes de carbone.
R1 désigne un radical hydroxyalkyle en C₁-C₄, de préférence hydroxyéthyle,
A, A1, A2 , identiques ou différents, désignent un radical alkylène divalent linéaire ou ramifié en C₁-C₁₀, de préférence en C₁-C₃
X désigne un atome d'hydrogène, un cation minéral ou organique.

5. Composition selon la revendication 4, **caractérisée par le fait que** R désigne de préférence un radical alkyle ou alkényle mono ou polyinsaturé comportant de 5 à 29 atomes de carbone et de préférence de 7 à 22 atomes de carbone et encore plus particulièrement de 9 à 17 atomes de carbone.

6. Composition selon l'une quelconque des revendications 4 ou 5, **caractérisée par le fait que** A et A2 désignent -CH₂CH₂-.

7. Composition selon l'une quelconque des revendications 4 à 6, **caractérisée par le fait que** A1 désigne -CH₂-.

8. Composition selon l'une quelconque des revendications 4 à 7, **caractérisée par le fait que** le cation minéral ou organique est choisi parmi celui d'un métal alcalin (par exemple Na⁺, K⁺) , celui d'un métal alcalinoterreux (Mg²⁺, Ca²⁺) , l'ion NH₄⁺ , les ions ammoniums issus des aminoacides basiques ou des amino-alcools.

9. Composition selon l'une quelconque des revendications 1 à 8, **caractérisée par le fait que** ledit tensioactif amphotère est choisi parmi les sels de Cocoyl amphohydroxypropyl sulfonate et les sels de palmitoyl amphohydroxypropyl sulfonate.

10. Composition selon l'une quelconque des revendications 1 à 9, **caractérisée en ce que** le ou les tensioactifs amphotères alcoylamphohydroxyalkylsulfonates sont présents dans les compositions à une concentration comprise entre 1 et 30 % en poids, de préférence entre 1,5 et 15% en poids par rapport au poids total de la composition.

11. Composition selon l'une quelconque des revendications 1 à 10, **caractérisée en ce que** le ou les composés de type céramide sont présents à une concentration comprise entre 0,0001 % et 10 % en poids par rapport au poids total de la composition, de préférence entre 0,005 % et 5 % en poids et en particulier entre 0,01 % et 3 % en poids.

12. Composition selon l'une quelconque des revendications 1 à 11, **caractérisée par le fait qu'**elle comprend en outre au moins un agent tensioactif additionnel choisi parmi les tensioactifs anioniques, cationiques, non ioniques, amphotères et leurs mélanges.

13. Compositions selon la revendication 12, **caractérisées par le fait que** le ou les agents tensioactifs additionnels sont présents à une concentration comprise entre 0,1% et 60% en poids, de préférence entre 3% et 40% en poids, et encore plus préférentiellement entre 5% et 30% en poids, par rapport au poids total de la composition.

14. Composition selon l'une quelconque des revendications 1 à 13, **caractérisée par le fait qu'**elle comprend en outre au moins un additif choisi parmi les épaississants, les parfums, les agents nacrants et/ou opacifiants, les conservateurs, les filtres solaires, les agents tensioactifs cationiques, les polymères anioniques ou non ioniques ou cationiques ou amphotères, les protéines, les hydrolysats de protéines, les acides gras à chaînes linéaires ou ramifiées en C₁₆-C₄₀ tels que l'acide méthyl-18 eicosanoique, les hydroxyacides, les vitamines, le panthénol, les silicones volatiles ou non volatiles et les esters gras.

15. Composition selon la revendication 14, **caractérisée par le fait que** lesdits polymères cationiques sont choisis parmi :
- les homopolymères de sel de diallyldiméthylammonium et les copolymères de sel de diallyldiméthylammonium et d'acrylamide,
- les dérivés d'éther de cellulose quaternaires
- les polysaccharides cationiques non cellulosiques
- les polymères comprenant des motifs récurrents répondant à la formule : 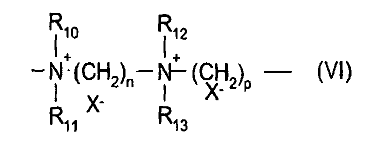
dans laquelle R₁₀, R₁₁, R₁₂ et R_{13,} identiques ou différents, désignent un radical alkyle ou hydroxyalkyle ayant de 1 à 4 atomes de carbone, n et p sont des nombres entiers variant de 2 à 20 environ et, X- est un anion dérivé d'un acide minéral ou organique.

16. Composition selon la revendication 14, **caractérisée par le fait que** les agents nacrants et/ou opacifiants sont choisis parmi les oxydes de titane, les palmitates de sodium ou de magnésium, les stéarates et hydroxystéarates de sodium ou de magnésium, les dérivés acylés à chaîne grasse tels que les monostéarates ou distéarates d'éthylène glycol ou de polyéthylèneglycol, les éthers à chaînes grasses ( par exemple en C8-C30) tels que par exemple le distéaryléther ou le 1-(hexadécyloxy)-2-octadécanol, les cyclodextrines, les alcools gras tels que les alcools cétylique, stéarylique et béhénylique.

17. Composition selon l'une quelconque des revendications 1 à 16, **caractérisée par le fait qu'**elle se présente sous forme de shampooing, d'après-shampooing, de composition pour la permanente, le défrisage, la coloration ou la décoloration des cheveux, de composition à rincer à appliquer entre les deux étapes d'une permanente ou d'un défrisage, de composition lavante pour la peau.

18. Utilisation d'une composition telle que définie dans l'une quelconque des revendications 1 à 17 pour le lavage des matières kératiniques telles que les cheveux.

19. Procédé de traitement des matières kératiniques, telles que les cheveux, **caractérisé en ce qu'**il consiste à appliquer sur lesdites matières une composition cosmétique selon l'une des revendications 1 à 17, puis à effectuer éventuellement un rinçage à l'eau.

20. Utilisation d'un tensioactif amphotère alcoylampho hydroxyalkyl sulfonate ou ses sels pour améliorer la fixation des composés de type céramide sur et/ou dans les matières kératiniques.

21. Utilisation d'une composition selon l'une des revendications 1 à 17 pour améliorer le démêlage ou le lissage des cheveux, ou pour apporter du volume, de la légèreté, de la douceur, de la souplesse, de la malléabilité aux cheveux.

22. Utilisation d'un tensioactif amphotère alcoylamphohydroxyalkylsulfonate ou ses sels dans une composition cosmétique contenant au moins un composé de type céramide pour améliorer le démêlage ou le lissage des cheveux, ou pour apporter du volume, de la légèreté, de la douceur, de la souplesse ou de la malléabilité aux cheveux.

## Claims

1. Cosmetic composition, **characterized in that** it comprises, in a cosmetically acceptable medium, at least one amphoteric surfactant chosen from alkylamphohydroxyalkylsulphonate compounds and salts thereof and at least one compound of ceramide type.

2. Composition according to Claim 1, **characterized in that** the compound of ceramide type has formula (I) below: 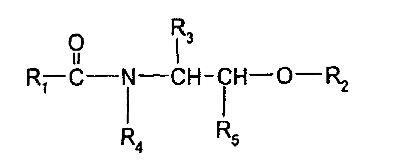 in which:
- R₁ denotes:
- either a linear or branched, saturated or unsaturated C₁-C₅₀, preferably C₅-C₅₀, hydrocarbon-based radical, it being possible for this radical to be substituted with one or more hydroxyl groups optionally esterified with an acid R₇COOH, R₇ being a linear or branched, saturated or unsaturated, optionally monohydroxylated or polyhydroxylated C₁-C₃₅ hydrocarbon-based radical, it being possible for the hydroxyl(s) of the R₇ radical to be esterified with a linear or branched, saturated or unsaturated, optionally monohydroxylated or polyhydroxylated C₁-C₃₅ fatty acid;
- or a radical R"-(NR°-CO)-R', where R° denotes a hydrogen atom or a monohydroxylated or polyhydroxylated, preferably monohydroxylated, C₁-C₂₀ hydrocarbon-based radical, and R' and R" are hydrocarbon-based radicals, the sum of the carbon atoms of which ranges from 9 to 30, R' being a divalent radical;
- or a radical R₈-O-CO-(CH₂)ₚ, where R₈ denotes a C₁-C₂₀ hydrocarbon-based radical, and p is an integer ranging from 1 to 12;
- R₂ is chosen from a hydrogen atom, a radical of saccharide type, in particular a (glycosyl)ₙ, (galactosyl)ₘ or sulphogalactosyl radical, a sulphate or phosphate residue, a phosphorylethylamine radical and a phosphorylethylammonium radical, in which n is an integer ranging from 1 to 4 and m is an integer ranging from 1 to 8;
- R₃ denotes a hydrogen atom or a saturated or unsaturated, monohydroxylated, polyhydroxylated or nonhydroxylated, C₁-C₃₃ hydrocarbon-based radical, it being possible for the hydroxyl(s) to be esterified with an inorganic acid or an acid R₇COOH, R₇ having the same meanings as above, it being possible for the hydroxyl(s) to be etherified with a (glycosyl)ₙ, (galactosyl)ₘ, sulphogalactosyl, phosphorylethylamine or phosphorylethylammonium radical, it also being possible for R₃ to be substituted with one or more C₁-C₁₄ alkyl radicals;
preferably, R₃ denotes a C₁₅-C₂₆ α-hydroxyalkyl radical, the hydroxyl group being optionally esterified with a C₁₆-C₃₀ α-hydroxy acid;
- R₄ denotes a hydrogen atom, a methyl radical, an ethyl radical, a linear or branched, saturated or unsaturated, optionally hydroxylated C₃-C₅₀ hydrocarbon-based radical or a radical -CH₂-CHOH-CH₂-O-R₆ in which R₆ denotes a C₁₀-C₂₆ hydrocarbon-based radical or a radical R₈-O-CO-(CH₂)ₚ, R₈ denotes a C₁-C₂₀ hydrocarbon-based radical and p is an integer ranging from 1 to 12;
- R₅ denotes a hydrogen atom or a linear or branched, saturated or unsaturated, optionally monohydroxylated or polyhydroxylated C₁-C₃₀ hydrocarbon-based radical, it being possible for the hydroxyl(s) to be etherified with a (glycosyl)ₙ, (galactosyl)ₘ, sulphogalactosyl, phosphorylethylamine or phosphorylethylammonium radical,
with the proviso that, when R₃ and R₅ denote hydrogen or when R₃ denotes hydrogen and R₅ denotes methyl, then R₄ does not denote a hydrogen atom, a methyl radical or an ethyl radical.

3. Composition according to either one of Claims 1 and 2, **characterized in that** the compound of ceramide type is chosen from the group consisting of:
- 2-N-linoleoylaminooctadecane-1,3-diol,
- 2-N-oleoylaminooctadecane-1,3-diol,
- 2-N-palmitoylaminooctadecane-1,3-diol,
- 2-N-stearoylaminooctadecane-1,3-diol,
- 2-N-behenoylaminooctadecane-1,3-diol,
- 2-N-[2-hydroxypalmitoyl]aminooctadecane-1,3-diol,
- 2-N-stearoylaminooctadecane-1,3,4-triol, and in particular N-stearoylphytosphingosine,
- 2-N-palmitoylaminohexadecane-1,3-diol,
- bis(N-hydroxyethyl-N-cetyl)malonamide,
- cetylic acid N-(2-hydroxyethyl)-N-(3-cetyloxy-2-hydroxypropyl)amide,
- N-docosanoyl-N-methyl-D-glucamine,
or mixtures of these compounds.

4. Composition according to any one of Claims 1 to 3, **characterized in that** said amphoteric surfactant is chosen from the alkylamphohydroxyalkylsulphonate compounds that may have the following formula (II): 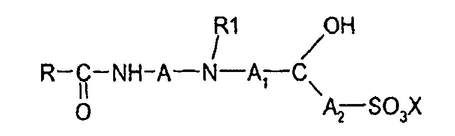 in which:
R denotes a linear or branched, saturated or unsaturated, hydrocarbon-based radical containing from 5 to 29 carbon atoms,
R1 denotes a C₁-C₄ hydroxyalkyl radical, preferably hydroxyethyl,
A, A1 and A2, which may be identical or different, denote a linear or branched C₁-C₁₀, preferably C₁-C₃, divalent alkylene radical,
X denotes a hydrogen atom or an inorganic or organic cation.

5. Composition according to Claim 4, **characterized in that** R preferably denotes a monounsaturated or polyunsaturated alkyl or alkenyl radical containing from 5 to 29 carbon atoms, and preferably from 7 to 22 carbon atoms, and even more particularly from 9 to 17 carbon atoms.

6. Composition according to either one of Claims 4 and 5, **characterized in that** A and A2 denote -CH₂CH₂-.

7. Composition according to any one of Claims 4 to 6, **characterized in that** A1 denotes -CH₂-.

8. Composition according to any one of Claims 4 to 7, **characterized in that** the inorganic or organic cation is chosen from that of an alkaline metal (for example, Na⁺, K⁺), that of an alkaline earth metal (Mg²⁺, Ca²⁺) , the NH₄⁺ ion, and ammonium ions derived from basic amino acids or from amino alcohols.

9. Composition according to any one of Claims 1 to 8, **characterized in that** said amphoteric surfactant is chosen from cocoyl amphohydroxypropyl sulphonate salts and palmitoyl amphohydroxypropyl sulphonate salts.

10. Composition according to any one of Claims 1 to 9, **characterized in that** the alkylamphohydroxyalkylsulphonate amphoteric surfactant(s) is (are) present in the compositions at a concentration of between 1% and 30% by weight, preferably between 1.5% and 15% by weight, relative to the total weight of the composition.

11. Composition according to any one of Claims 1 to 10, **characterized in that** the compound(s) of ceramide type is (are) present at a concentration of between 0.0001% and 10% by weight relative to the total weight of the composition, preferably between 0.005% and 5% by weight, and in particular between 0.01% and 3% by weight.

12. Composition according to any one of Claims 1 to 11, **characterized in that** it also comprises at least one additional surfactant chosen from anionic, cationic, non-ionic and amphoteric surfactants, and mixtures thereof.

13. Compositions according to Claim 12, **characterized in that** the additional surfactant(s) is (are) present at a concentration of between 0.1% and 60% by weight, preferably between 3% and 40% by weight, and even more preferably between 5% and 30% by weight, relative to the total weight of the composition.

14. Composition according to any one of Claims 1 to 13, **characterized in that** it also comprises at least one additive chosen from thickeners, fragrances, pearlescent agents and/or opacifiers, preserving agents, sunscreens, cationic surfactants, anionic, non-ionic, cationic or amphoteric polymers, proteins, protein hydrolysates, C₁₆-C₄₀ linear- or branched-chain fatty acids such as 18-methyleicosanoic acid, hydroxy acid, vitamins, panthenol, volatile or non-volatile silicones, and fatty esters.

15. Composition according to Claim 14, **characterized in that** said cationic polymers are chosen from:
- diallyldimethylammonium salt homopolymers and copolymers of a diallyldimethylammonium salt and of acrylamide,
- quaternary cellulose ether derivatives,
- non-cellulosic cationic polysaccharides,
- polymers comprising repeating units corresponding to the formula: 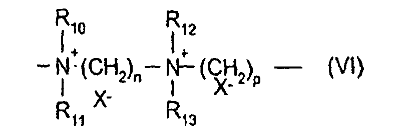
in which R₁₀, R₁₁, R₁₂ and R₁₃, which may be identical or different, denote an alkyl or hydroxyalkyl radical containing from 1 to 4 carbon atoms, n and p are integers ranging approximately from 2 to 20, and X⁻ is an anion derived from an inorganic or organic acid.

16. Composition according to Claim 14, **characterized in that** the pearlescent agents and/or opacifiers are chosen from titanium oxides, sodium palmitate, magnesium palmitate, sodium stearate or hydroxystearate, magnesium stearate or hydroxystearate, fatty-chain acylated derivatives such as ethylene glycol monostearates or distearates or polyethylene glycol monostearates or distearates, fatty-chain ethers (for example, C₈-C₃₀) such as, for example, distearyl ether or 1-(hexadecyloxy)-2-octadecanol, cyclodextrins, and fatty alcohols such as cetyl alcohol, stearyl alcohol and behenyl alcohol.

17. Composition according to any one of Claims 1 to 16, **characterized in that** it is in the form of a shampoo, a conditioner, a permanent-waving, hair-straightening, dyeing or bleaching composition for the hair, a rinse-out composition to be applied between the two steps of a permanent wave or a hair-straightening operation, or a washing composition for the skin.

18. Use of a composition as defined in any one of Claims 1 to 17, for washing keratin materials such as the hair.

19. Process for treating keratin materials, such as the hair, **characterized in that** it consists in applying to said materials a cosmetic composition according to one of Claims 1 to 17, and then in optionally rinsing it out with water.

20. Use of an alkylamphohydroxyalkylsulphonate amphoteric surfactant or salts thereof, for improving the fixing of compounds of ceramide type on and/or in keratin materials.

21. Use of a composition according to one of Claims 1 to 17, for improving the disentangling or the smoothing of the hair, or for imparting volume, lightness, softness, suppleness or manageability on the hair.

22. Use of an alkylamphohydroxyalkylsulphonate amphoteric surfactant or salts thereof, in a cosmetic composition containing at least one compound of ceramide type, for improving the disentangling or the smoothing of the hair, or for imparting volume, lightness, softness, suppleness or manageability on the hair.

## Patentansprüche

1. Kosmetische Zusammensetzung, **dadurch gekennzeichnet, dass** sie in einem kosmetisch akzeptablen Medium mindestens einen amphoteren grenzflächenaktiven Stoff, der unter den Alkoylamphohydroxyalkylsulfonaten und ihren Salzen ausgewählt ist, und mindestens eine Verbindung vom Ceramidtyp enthält.

2. Zusammensetzungen nach Anspruch 1, **dadurch gekennzeichnet, dass** die Verbindung vom Ceramidtyp der folgenden Formel (I) entspricht: 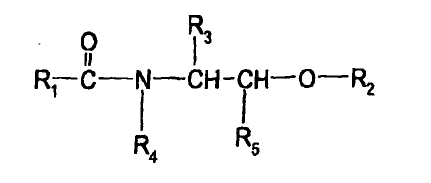 worin in der Formel:
- R₁ bedeutet:
- entweder eine geradkettige oder verzweigte, gesättigte oder ungesättigte C₁₋₅₀-Kohlenwasserstoffgruppe und vorzugsweise C₅₋₅₀-Kohlenwasserstoffgruppe, wobei diese Gruppe mit einer oder mehreren Hydroxygruppen substituiert sein kann, die gegebenenfalls mit einer Säure R₇COOH verestert sein können, wobei R₇ eine gesättigte oder ungesättigte, geradkettige oder verzweigte C₁₋₃₅-Kohlenwasserstoffgruppe ist, die gegebenenfalls ein- oder mehrfach hydroxyliert ist, wobei die Hydroxygruppe(n) der Gruppe R₇ mit einer gesättigten oder ungesättigten, geradkettigen oder verzweigten, gegebenenfalls ein- oder mehrfach hydroxylierten C₁₋₃₅-Fettsäure verestert sein kann (können);
- oder eine Gruppe R"-NR°-CO-R', wobei R° ein Wasserstoffatom oder eine C₁₋₂₀-Kohlenwasserstoffgruppe mit einer oder mehreren Hydroxygruppen und vorzugsweise einer Hydroxygruppe bedeutet und R' und R" Kohlenwasserstoffgruppen sind, deren Summe an Kohlenstoffatomen im Bereich von 9 bis 30 liegt, wobei R' eine zweiwertige Gruppe ist;
- oder eine Gruppe R₈-O-CO-(CH₂)ₚ, wobei R₈ eine C₁₋₂₀-Kohlenwasserstoffgruppe ist und p eine ganze Zahl im Bereich von 1 bis 12 bedeutet;
- R₂ ist ausgewählt unter einem Wasserstoffatom, einer Gruppe vom Saccharidtyp, insbesondere (Glycosyl)ₙ, (Galactosyl),ₘ oder Sulfogalactosyl, einer Sulfat- oder Phosphatgruppe, einer Phosphorylethylamingruppe und einer Phosphorylethylammoniumgruppe, wobei n eine ganze Zahl im Bereich von 1 bis 4 und m eine ganze Zahl im Bereich von 1 bis 8 ist;
- R₃ bedeutet Wasserstoff oder eine gesättigte oder ungesättigte, ein- oder mehrfach hydroxylierte oder nicht hydroxylierte C₁₋₃₃-Kohlenwasserstoffgruppe, wobei die Hydroxygruppe(n) mit einer anorganischen Säure oder einer Säure R₇COOH verestert sein kann (können), wobei R₇ die oben angegebenen Bedeutungen aufweist, oder die Hydroxygruppe(n) mit (Glycosyl)n, (Galactosyl)m, Sulfogalactosyl, Phosphorylethylamin oder einer Phosphorylethylammonium verethert sein kann (können) und wobei die Gruppe R₃ mit einer oder mehreren C₁₋₁₄-Alkylgruppen substituiert sein kann;
R₃ bedeutet vorzugsweise eine α-Hydroxyalkylgruppe mit 15 bis 26 Kohlenstoffatomen, wobei die Hydroxygruppe gegebenenfalls mit einer α-Hydroxysäure mit 16 bis 30 Kohlenstoffatomen verestert sein kann;
- R₄ Wasserstoff, Methyl, Ethyl, eine gesättigte oder ungesättigte, geradkettige oder verzweigte, gegebenenfalls hydroxylierte C₃₋₅₀-Kohlenwasserstoffgruppe, eine Gruppe -CH₂-CHOH-CH₂-O-R₆, wobei R₆ eine C₁₀₋₂₆-Kohlenwasserstoffgruppe bedeutet, oder eine Gruppe R₈-O-CO-(CH₂)ₚ, wobei R₈ eine C₁₋₂₀-Kohlenwasserstoffgruppe bedeutet und p eine ganze Zahl im Bereich von 1 bis 12 ist;
- R₅ Wasserstoff oder eine gesättigte oder ungesättigte, geradkettige oder verzweigte, gegebenenfalls ein- oder mehrfach hydroxylierte C₁₋₃₀-Kohlenwasserstoffgruppe, wobei die Hydroxygruppe(n) mit (Glycosyl)ₙe, (Galactosyl)ₘ, Sulfogalactosyl, Phosphorylethylamin oder einer Phosphorylethylammonium verethert sein kann (können);
mit der Maßgabe, dass R₄ nicht Wasserstoff, Methyl oder Ethyl bedeutet, wenn R₃ und R₅ Wasserstoff bedeuten oder wenn R₃ Wasserstoff und R₅ Methyl bedeutet.

3. Zusammensetzungen nach einem der Ansprüche 1 oder 2, **dadurch gekennzeichnet, dass** die Verbindung vom Ceramidtyp ausgewählt ist unter:
- 2-N-Linoleoylamino-octadecan-1,3-diol,
- 2-N-Oleoylamino-octadecan-1,3-diol,
- 2-N-Palmitoylamino-octadecan-1,3-diol,
- 2-N-Stearoylamino-octadecan-1,3-diol,
- 2-N-Behenoylamino-octadecan-1,3-diol
- 2-N-[2-Hydroxy-palmitoyl]-amino-octadecan-1,3-diol,
- 2-N-Stearoylamino-octadecan-1,3,4-triol und insbesondere N-Stearoyl-phytosphingosin,
- 2-N-Palmitoylamino-hexadecan-1,3-diol,
- (Bis(N-hydroxyethyl-N-cetyl)-malonamid),
- N-(2-Hydroxyethyl)-N-(3-cetyloxy-2-hydroxypropyl)-cetylsäureamid,
- N-Docosanyl-N-methyl-D-glucamin,
- oder den Gemischen dieser Verbindungen.

4. Zusammensetzung nach einem der Ansprüche 1 bis 3, **dadurch gekennzeichnet, dass** der amphotere grenzflächenaktive Stoff unter den Alkoylamphohydroalkylsulfonaten ausgewählt ist, die die folgende Formel (I) aufweisen können: 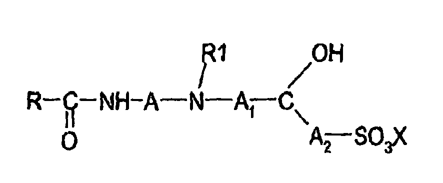 worin bedeuten:
R eine geradkettige oder verzweigte, gesättigte oder ungesättigte Kohlenwasserstoffgruppe mit 5 bis 29 Kohlenstoffatomen,
R1 eine C₁₋₄-Hydroxyalkylgruppe, vorzugsweise Hydroxyethyl,
A, A1 und A2, die gleich oder voneinander verschieden sind, eine geradkettige oder verzweigte, zweiwertige C₁₋₁₀-Alkylengruppe und vorzugsweise C₁₋₃-Alkylengruppe,
X ein Wasserstoffatom, ein organisches oder anorganisches Kation.

5. Zusammensetzung nach Anspruch 4, **dadurch gekennzeichnet, dass** R vorzugsweise eine Alkylgruppe oder eine ein- oder mehrfach ungesättigte Alkenylgruppe mit 5 bis 29 Kohlenstoffatomen, vorzugsweise 7 bis 22 Kohlenstoffatomen und insbesondere 9 bis 17 Kohlenstoffatomen bedeutet.

6. Zusammensetzung nach einem der Ansprüche 4 oder 5, **dadurch gekennzeichnet, dass** A und A2 -CH₂CH₂- bedeuten.

7. Zusammensetzung nach einem der Ansprüche 4 bis 6, **dadurch gekennzeichnet, dass** A1 -CH₂- bedeutet.

8. Zusammensetzung nach einem der Ansprüche 4 bis 7, **dadurch gekennzeichnet, dass** das anorganische oder organische Kation unter den Kationen eines Alkalimetalls (beispielsweise Na⁺, K⁺), den Kationen eines Erdalkalimetalls (beispielsweise Mg²⁺, Ca²⁺), dem Ion NH₄⁺ und den Ammoniumionen ausgewählt ist, die von basischen Aminosäuren oder Aminoalkoholen stammen.

9. Zusammensetzung nach einem der Ansprüche 1 bis 8, **dadurch gekennzeichnet, dass** der amphotere grenzflächenaktive Stoff unter den Salzen von Cocoylamphohydroxypropylsulfonat und den Salzen von Palmitoylamphohydroxypropylsulfonat ausgewählt ist.

10. Zusammensetzung nach einem der Ansprüche 1 bis 9, **dadurch gekennzeichnet, dass** das oder die amphoteren grenzflächenaktiven Alkoylamphohydroxyalkylsulfonate in den Zusammensetzungen in einer Konzentration von 1 bis 30 Gew.-% und vorzugsweise 1,5 bis 15 Gew.-%, bezogen auf das Gesamtgewicht der Zusammensetzung, enthalten sind.

11. Zusammensetzung nach einem der Ansprüche 1 bis 10, **dadurch gekennzeichnet, dass** die Verbindungen(en) vom Ceramidtyp in einer Konzentration von 0,0001 bis 10 Gew.-%, bezogen auf das Gesamtgewicht der Zusammensetzung, vorzugsweise 0,005 bis 5 Gew.-% und insbesondere 0,01 bis 3 Gew.-% enthalten sind.

12. Zusammensetzung nach einem der Ansprüche 1 bis 11, **dadurch gekennzeichnet, dass** sie ferner mindestens einen zusätzlichen grenzflächenaktiven Stoff enthält, der unter den anionischen, kationischen, nichtionischen oder amphoteren grenzflächenaktiven Stoffen und deren Gemischen ausgewählt ist.

13. Zusammensetzungen nach Anspruch 12, **dadurch gekennzeichnet, dass** der oder die zusätzlichen grenzflächenaktiven Stoffe in einer Konzentration von 0,1 bis 60 Gew.-%, vorzugsweise 3 bis 40 Gew.-% und noch bevorzugter 5 bis 30 Gew.-%, bezogen auf das Gesamtgewicht der Zusammensetzung, vorliegen.

14. Zusammensetzung nach einem der Ansprüche 1 bis 13, **dadurch gekennzeichnet, dass** sie ferner mindestens einen Zusatzstoff enthält, der unter den Verdickungsmitteln, Parfums, Perlglanzpigmenten und/oder Trübungsmitteln, Konservierungsmitteln, Sonnenschutzfiltern, kationischen grenzflächenaktiven Stoffen, anionischen oder nichtionischen, oder kationischen oder amphoteren Polymeren, Proteinen, Proteinhydrolysaten, Fettsäuren mit geraden oder verzweigten C₁₆-₄₀-Ketten, beispielsweise 18-Methyleicosansäure, Hydroxysäuren, Vitaminen, Panthenol, flüchtigen oder nichtflüchtigen Siliconen und Fettsäureestern ausgewählt ist.

15. Zusammensetzung nach Anspruch 145, **dadurch gekennzeichnet, dass** die kationischen Polymere ausgewählt sind unter:
- Homopolymeren von Diallyldimethylammoniumsalzen und Copolymeren von Diallyldimethylammoniumsalzen und Acrylamid,
- quartären Celluloseeetherderivaten,
- kationischen Polysacchariden, die nicht auf Cellulose basieren,
- Polymeren mit Wiederholungseinheiten der folgenden Formel: 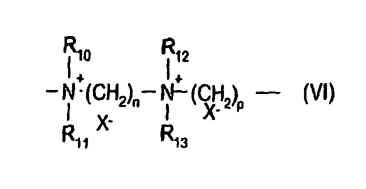
worin Rio, R₁₁, R₁₂ und R₁₃, die gleich oder verschieden sind, eine Alkyl- oder Hydroxyalkylgruppe mit 1 bis 4 Kohlenstoffatomen bedeuten, n und p ganze Zahlen im Bereich von etwa 2 bis 20 bedeuten, und X- ein Anion ist, das von einer anorganischen oder organischen Säure abgeleitet ist.

16. Zusammensetzung nach Anspruch 14, **dadurch gekennzeichnet, dass** die Perlglanzpigmente und/oder Trübungsmittel unter den Titanoxiden, Natrium- oder Magnesiumpalmitat, Natrium- oder Magnesiumstearat und Natrium- oder Magnesiumhydroxystearat, Acylderivaten mit Fettkette, wie Mono- oder Distearaten von Ethylenglycol oder Polyethylenglycol, Ethern mit Fettkette (beispielsweise C8-C30), wie dem Distearylether oder 1-(Hexadecyloxy)-2-octadodecanol, Cyclodextrinen, Fettalkoholen, wie Cetylalkohol, Stearylalkohol und Behenylalkohol, ausgewählt sind.

17. Zusammensetzung nach einem der Ansprüche 1 bis 16, **dadurch gekennzeichnet, dass** sie als Haarwaschmittel, Produkt, das nach der Haarwäsche angewandt wird, Zusammensetzung für dauerhafte Verformungen, Entkräuselungen, Färbungen oder Entfärbungen der Haare, Zusammensetzung, die ausgespült wird und zwischen den beiden Schritten einer Dauerwelle oder Entkräuselung aufgetragen wird, oder reinige Zusammensetzung für die Haut vorliegt.

18. Verwendung einer Zusammensetzung nach einem der Ansprüche 1 bis 17 zur Reinigung von Keratinsubstanzen, wie zum Waschen der Haare.

19. Verfahren zur Behandlung von Keratinsubstanzen, wie Haaren, **dadurch gekennzeichnet, dass** auf die Keratinsubstanzen eine kosmetische Zusammensetzung nach einem der Ansprüche 1 bis 17 aufgetragen und anschließend gegebenenfalls mit Wasser gespült wird.

20. Verwendung eines amphoteren grenzflächenaktiven Alkoylamphohydroxyalkylsulfonats oder seiner Salze, um die Fixierung von Verbindungen vom Ceramidtyp an und/oder in Keratinsubstanzen zu verbessern.

21. Verwendung einer Zusammensetzung nach einem der Ansprüche 1 bis 17, um die Kämmbarkeit oder Glatte der Haare zu verbessern oder für mehr Volumen, Leichtigkeit, Weichheit, Geschmeidigkeit und Formbarkeit der Haare zu sorgen.

22. Verwendung eines amphoteren grenzflächenaktiven Alkoylamphohydroxyalkylsulfonats oder seiner Salze in einer kosmetischen Zusammensetzung, die mindestens eine Verbindung vom Ceramidtyp enthält, um die Kämmbarkeit oder Glätte der Haare zu verbessern oder für mehr Volumen, Leichtigkeit, Weichheit, Geschmeidigkeit oder Formbarkeit der Haare zu sorgen.
